# EUROPÄISCHE PATENTSCHRIFT

(11) **EP 2 020 227 B1**
(45) Veröffentlichungstag und Bekanntmachung des Hinweises auf die Patenterteilung: **15.08.2012**
(21) Anmeldenummer: 08017772.8
(22) Anmeldetag: 05.01.2006
(51) Int. Cl.: A61K 8/64, A61K 8/73, A61Q 5/02, A61Q 5/12, A61Q 19/00, A61Q 19/10

(54) **Kationische Cellulosederivate in Kosmetika**
Cationic cellulose derivatives for cosmetics
Dérivés de cellulose cationiques en cosmétique

(30) Priorität: 14.02.2005 DE 102005006837
(43) Veröffentlichungstag der Anmeldung: 04.02.2009
(62) Teilanmeldung aus: 06000160.9
(73) Patentinhaber: Henkel AG & Co. KGaA, 40589 Düsseldorf (DE)
(72) Erfinder: Scheunemann, Volker, 21339 Lüneburg (DE); Schröder, Thomas, 22395 Hamburg (DE); Schulze Zur Wiesche, Erik, 20144 Hamburg (DE); Goddinger, Dieter, 25336 Klein Nordende (DE)

(56) Entgegenhaltungen:
- WO-A-02/100360
- WO-A-2005/000903
- DE-A1- 4 207 046
- ANONYMOUS: "SoftCAT SL Polymers" INTERNET ARTICLE, [Online] Oktober 2004 (2004-10), XP002387806 Gefunden im Internet: URL:http://www.dow.com/ucc/amerchol/resour ce/324-00187.pdf> [gefunden am 2006-06-28]

## Beschreibung

Die Erfindung betrifft ein Haut- und Haarreinigungs- und -konditioniermittel auf der Basis eines hydrophob modifizierten, kationischen Cellulosepolymeren in der Kombination mit mindestens einem weiteren Haarpflegestoff, sowie die Verwendung dieses Mittels zur Verbesserung des Haar- und des Hautzustandes.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Nicht zuletzt durch die starke Beanspruchung der Haare, beispielsweise durch das Färben oder Dauerwellen, durch die Reinigung der Haare mit Shampoos und durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt, der Griff und die Fülle des Haares optimiert, oder die Haare vor erhöhtem Spliß geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt, der Griff und die Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten zur Reinigung der Haare zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger benötigt wird.

Die zur Verfügung stehenden Wirkstoffe sowohl für separate Nachbehandlungsmittel als auch für Kombinationspräparate wirken im allgemeinen bevorzugt an der Haarobeffläche. So sind Wirkstoffe bekannt, welche dem Haar Weichheit, Glätte, Glanz, Halt, Fülle, Griff oder bessere Naß- oder Trockenkämmbarkeiten verleihen oder dem Spliß vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflußt werden kann.

Die bekannten Wirkstoffe können jedoch nicht alle Bedürfnisse in ausreichendem Maße abdecken. Es besteht daher weiterhin ein Bedarf nach Wirkstoffen bzw.

Wirkstoffkombinationen für haarkosmetische Mittel mit sehr guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen.

Die herausragende Wirkung kationischer Cellulosepolymere in kosmetischen Haar-reinigungs-und Konditioniermitteln ist seit langem bekannt. Beispielsweise in Cosmetics & Toiletries 2004, 156, wird die Verbesserung der Nass- und Trockenkämmbarkeit, des Haargriffs und des äußeren Erscheinungsbildes der Haare durch die Einführung dieser Polymertypen in Haarreinigungs- und -konditioniermitteln beschrieben.

Aus der EP 1 009 365 B1 ist bekannt, dass kationische Guar-Polymere durch die Einführung hydrophober Gruppen in ihrer Wirkung verstärkt werden und einem Haarbehandlungsmittel auf der Basis eines solchen Polymeren in der Kombination mit einem Silikon eine signifikant verbesserte Pflegeleistung verleihen.

In dem Artikel XP002387806, erhältlich im Internet unter der Adresse http://www.dow.com/ucc/amerchol/resour ce/324-00187.pdf, sowie in der WO 2005/000903 A1 werden hydrophob modifizierte und kationisch substituierte Polymere beschrieben, die die Trockenkämmbarkeit der Haare und die Silikonabscheidung verbessern, sowie unerwünschte built-up-Effekte beseitigen.

WO 2002/100360 A1 betrifft die Kombination hydrophob modifizierter Cellulosederivate mit kationischen Tensiden und hoch schmelzenden Fettstoffen zur optimalen Volumenerhaltung sowie zur Steigerung der Weichheit und des Griffs der Haare.

In der DE 42 07 046 A1 werden kationische Polymere mit pflanzlichen Proteinhydrolysaten in einer anionischen Tensidbasis kombiniert. Dadurch konnten die Haarstruktur, die Fülle, das Volumen und der Glanz des behandelten Haares verbessert werden.

Vollkommen überraschend wurde nun gefunden, dass eine Wirkstoffkombination aus einem neuartigen, kationischen, hydrophob modifizierten Cellulosepolymeren und einem weiteren Haarpflegestoff in Form eines Proteinhydrolysats sich als Pflegekomponente in Haarbehandfungsmitteln in hohem Maße eignet und insbesondere hinsichtlich Glätte, Weichheit, Haargriff und Kämmbarkeit eine verbesserte Leistung zeigt.

Gegenstand der Erfindung ist daher ein Reinigungs- und -konditioniermittel für Haut und Haare, enthaltend - bezogen auf sein Gewicht -
a) 0,001 bis 10 Gew.-% mindestens eines quaternisierten Hydroxyethylcellulosepolymers, das mit Trimethylammoniumgruppen kationisch substituiert und mit Dimethyldodecylammoniumgruppen hydrophob modifiziert wurde, sowie
b) mindestens ein Proteinhydrolysat.

Die Polymere a) weisen bevorzugt einen kationischen Substitutionsgrad von 0,15 bis 0,25 und einen hydrophoben Substitutionsgrad (HS) von HS ≤ 0,01 auf.

Erfindungsgemäß bevorzugte Polymere a) weisen in einer dreiprozentigen, wässrigen Lösung bei einer Temperatur von 20 °C und einer Scherrate von 0,1/s eine Viskosität von 500 bis 900 Pas auf (gemessen mit dem schubspannungsgesteuerten Rotationsviskosimeter AR-1 000N der Firma Thermal Analysis, Platte-Kegel Messsystem mit 4 cm Durchmesser und einem Winkel von 1°, Spaltweite: 32).

Weiterhin beträgt die Fließgrenze erfindungsgemäß bevorzugter Polymere a) in einer dreiprozentigen, wässrigen Lösung bei einer Temperatur von 20 °C 100-200 Pa (gemessen im Oszillationsversuch mit einer konstanten Frequenz von 1 Hz und ansteigender Amplitude mit dem schubspannungsgesteuerten Rotationsviskosimeter AR-1000N der Firma Thermal Analysis, Platte-Kegel Messsystem mit 4 cm Durchmesser und einem Winkel von 1°, Spaltweite: 32).

Besonders geeignete Polymere a) sind die unter dem Handelsnamen Polymer SL (Polymer SL-5, Polymer SL-30, Polymer SL-60 und Polymer SL-100) von der Firma Dow Amerchol im Handel erhältlichen Polymertypen.

Erfindungsgemäß geeignete Vitamine, Pro-Vitamine und Vitaminvorstufen sind diejenigen, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A- Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅- Typs sind in den erfindungsgemäß verwendeten Mitteln bevorzugt in Mengen von 0,05-10 Gew.-%, bezogen auf das gesamte Mittel, enthalten. Mengen von 0,1-5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S,6aR)-2- Oxohexahydrothienol[3,4-d]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0, 0001 bis 1,0 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B, C und E.

Panthenol, Pantolacton, Pyridoxin und seine Derivate sowie Nicotinsäureamid und Biotin sind besonders bevorzugt.

Geeignete Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L- Methionin-S- Methylsulfoniumchlorid. Selbstverständlich können erfindungsgemäß auch β- Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.
Erfindungsgemäß können Proteinhydrolysate sowohl pflanzlichen als auch tierischen oder marinen oder synthetischen Ursprungs eingesetzt werden.
Tierische Proteinhydrolysate sind beispielsweise Elastin-, Kollagen-, Keratin- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.
Erfindungsgemäße Proteinhydrolysate pflanzlichen Ursprungs sind z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt) , Lexein® (Inolex), Hydrosoy® (Croda), Hydrolupin® (Croda), Hydrosesame® (Croda), Hydrotritium® (Croda) und Crotein® (Croda) erhältlich.
Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda) oder Crotein® (Croda) vertrieben.
Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n- Alkyl)-N-(2-hydroxy-3-chloro-n-propyl)- ammoniumhalogeniden durchgeführt. Weiterhin können die kationischen Proteinhydrolysate auch noch weiter derivatisiert sein. Als typische Als Beispiele für die erfindungsgemäßen kationischen Proteinhydrolysate und -derivate seien die unter den INCI- Bezeichnungen im "International Cosmetic Ingredient Dictionary and Handbook", (seventh edition 1997, The Cosmetic, Toiletry, and Fragrance Association 1101 17th Street, N. W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Selbstverständlich umfaßt die erfindungsgemäße Lehre alle isomeren Formen, wie cistrans-Isomere, Diastereomere und chirale Isomere.
Erfindungsgemäß ist es auch möglich, eine Mischung aus mehreren Proteinhydrolysaten (P) einzusetzen.

Besonders bevorzugt sind erfindungsgemäß die kationischen Proteinhydrolysate auf der Basis von Weizen, Soja, Seide und Kertain.

Die Proteinhydrolysate (P) sind in den Mitteln in Konzentrationen von 0,01 Gew.% bis zu 20 Gew.%, vorzugsweise von 0,05 Gew.% bis zu 15 Gew. % und ganz besonders bevorzugt in Mengen von 0,5 Gew.% bis zu 5 Gew.% enthalten.

Erfindungsgemäß geeignete Aminosäuren sind Lysin, Isoleucin, Tyrosin, Cystein, Cystin, Alanin, Serin, Tryptophan, Glutaminsäure, Threonin, Valin, Glycin, Prolin, Arginin und/oder Taurin. Sie werden in den erfindungsgemäßen Haarreinigungs- und - konditioniermitteln in einer Konzentration von 0,01 bis 5 Gew.-%, insbesondere in einer Konzentration von 0,05 bis 3 Gew.-%, eingesetzt.

Unter geeigneten Carbonsäuren im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C-Atomen in der Kette.
Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-, C2-C8-Hydroxyalkenyl-, Aminomethyl-, C2-C8- Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in α- Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.
Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, 4-Hydroxybenzoesäure, o,m,p- Phthalsäure, Naphthoesäure, Toluoylsäure, Hydratropasäure, Atropasäure, Salicylsäure, Zimtsäure, Isonicotinsäure, Nicotinsäure, Bicarbaminsäure, 4,4'-Dicyano-6, 6'-binicotinsäure, 8-Carbamoyloctansäure, 1,2,4-Pentantricarbonsäure, 2- Pyrrolcarbonsäure, 1,2,4,6,7-Napthalinpentaessigsäure, Malonaldehydsäure, 4- Hydroxy-phthalamidsäure, 1-Pyrazolcarbonsäure, Gallussäure oder Propantricarbonsäure.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Als geeignete organische Säuren im Sinne der Erfindung dienen auch Hydroxycarbonsäuren. Dabei handelt es sich sowohl um die Dihydroxy-, Trihydroxy- und Polyhydroxycarbonsäuren sowie um Hydroxycarbonsäureester und Mischungen aus Hydroxycarbonsäuren und deren Estern. Bevorzugte Hydroxycarbonsäuren und -ester sind beispielsweise Glycolsäure, Milchsäure, Äpfelsäure, Weinsäure oder Citronensäure und deren Ester. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8-22 C-Atomen, also z. B. Fettalkohole oder synthetische Fettalkohole. Dabei sind die Ester von C12- C15-Fettalkoholen besonders bevorzugt. Ester dieses Typs sind im Handel erhältlich, z. B. unter dem Warenzeichen Cosmacol® der EniChem, Augusta Industriale. Besonders bevorzugte Polyhydroxypolycarbonsäuren sind Polymilchsäure und Polyweinsäure sowie deren Ester.

Ganz besonders bevorzugte Carbonsäuren im Sinne der Erfindung sind Citronensäure, Bernsteinsäure, Salicylsäure, Milchsäure und/oder 4-Hydroxybenzoesäure sowie deren Salze und Derivate. Sie werden in den erfindungsgemäßen Mitteln in einer Menge von 0,01 bis 10 Gew.-%, insbesondere in einer Menge von 0,05 bis 5 Gew.-%, eingesetzt.

Unter erfindungsgemäß geeigneten, von a) verschiedenen, kationischen Polymeren sind Polymere zu verstehen, welche in der Haupt- und/oder Seitenkette Gruppen aufweisen, welche "temporär" oder "permanent" kationisch sein können. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

### Homopolymere der allgemeinen Formel (IV),

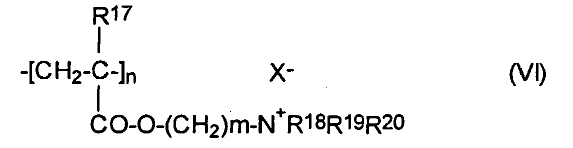

in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCl-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCl-Bezeichnung: Mineral Oil) und Tridecyl-polyoxypropylen-polyoxyethylen-ether (INCl-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCl-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (VI) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammonium-chlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Besonders bevorzugte, von a) verschiedene, kationische Polymere im Sinne der Erfindung sind quaternisierte Cellulose-Derivate, wie sie unter den Bezeichnungen Celquat^{®} und Polymer JR^{®} im Handel erhältlich sind, kationische Guar-Derivate, wie insbesondere die unter den Handelsnamen Cosmedia^{®}Guar und Jaguar^{®} vertriebenen Produkte sowie das unter dem Handelsnamen Salcare^{®} SC 96 vertriebene kationische Polymer.

Die kationischen Polymere sind in den erfindungsgemäßen Mitteln bevorzugt in Mengen von 0,01 bis 5 Gew.-%, bezogen auf das gesamte Mittel, enthalten. Mengen von 0,05 bis 4 Gew.-%, insbesondere von 0,1 bis 3 Gew.-%, sind besonders bevorzugt.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat- Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200°C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone gemäß der EP 0612759 B1.

Die nichtionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-%, insbesondere von 0,1 bis 3 Gew.- %, sind besonders bevorzugt.

Die erfindungsgemäße Wirkstoffkombination kann erfindungsgemäß sowohl in Haut- und Haarbehandlungsmitteln eingesetzt werden, die nach der Anwendung wieder aus- bzw. abgespült werden, als auch in solchen Haut- und Haarbehandlungsmitteln, die auf dem Haar oder der Haut verbleiben.

Zubereitungen, die üblicherweise nach der Anwendung aus dem Haar wieder ausgespült werden sind beispielsweise Shampoos, Spülungen und Konditionier- sowie Well- und Färbemittel. Produkte, die üblicherweise im Haar verbleiben, sind beispielsweise Haarfestiger, Haarkuren und Fönwellpräparate. Je nach Art der Zubereitung können neben den erfindungsgemäß obligatorischen Komponenten alle für den jeweiligen Anwendungszweck geeigneten, üblichen Hilfs- und Zusatzmittel eingesetzt werden.

Unter Produkten, die übelicherweise nach der Anwendung von der Haut wieder abgespült werden, sind erfindungsgemäß Flüssigseifen oder Duschbäder. Produkte, die üblicherweise auf der Haut verbleiben, sind Hautcremes.

In einer bevorzugten Ausführungsform der Erfindung wird die Wirkstoffkombination als Shampoo konfektioniert.

Diese enthalten neben der Basis Wasser üblicherweise anionische, nichtionische kationische, amphotere und/oder zwitterionische Tenside, kationische oder nichtionische Polymere, Perlglanzmittel, Parfümkomponenten, Mittel zur Einstellung des pH-Wertes, Farbstoffe, Konservierungsmittel, gegebenenfalls Ölkörper sowie Komponenten zur Einstellung der Viskosität.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 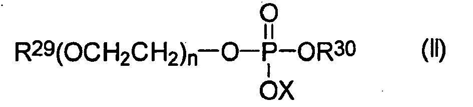 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglykolester der Formel (III),

   R³⁵CO(AlkO)ₙSO₃M (III)

   in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
   Monoglyceridsulfate und Monoglyceridethersulfate der Formel (IV), wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind 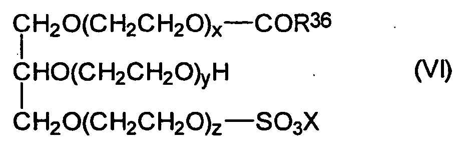 und in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (VIII) eingesetzt, in der R³⁶CO für einen linearen Acylrest mit 8 bis 18 Kohlenstoffatomen steht

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂-C₁₈ - Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (V)

   R³⁷CO-(OCH₂CH₂R³⁸)_{W}OR³⁹ (V)

   in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.
   Besonders bevorzugt sind solche Alkylpolyglykoside, bei denen R
   - im wesentlichen aus C₈- und C₁₀-Alkylgruppen,
   - im wesentlichen aus C₁₂- und C₁₄-Alkylgruppen,
   - im wesentlichen aus C₈- bis C₁₆-Alkylgruppen oder
   - im wesentlichen aus C₁₂- bis C₁₆-Alkylgruppen oder
   - im wesentlichen aus C₁₆ bis C₁₈-Alkylgruppen besteht.
   Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
   Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.
   Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide', -hydroxide oder -alkoholate als Katalysatoren verwendet werden. Die Verwendung von Produkten mit eingeengter Homologenverteilung kann bevorzugt sein.

Besonders bevorzugt werden als Tensidsystem eines oder mehrere Tenside aus der Gruppen der Alkylethersulfate, Aminoxide, Alkylpolyglycoside, Betaine, Alkyletherphosphate, Acyltaurine, Sulfobernsteinsäureester, Alkylethercarboxylate und/oder der Sorbitanester eingesetzt. Insbesondere bevorzugt sind Mittel, die Alkylethersulfate, Aminoxide, Alkylpolyglucoside und/oder Betaine enthalten.

Die Tenside werden in Mengen von 0,1 - 45 Gew.%, bevorzugt 1 - 30 Gew.% und ganz besonders bevorzugt von 1 - 15 Gew.%, bezogen auf das gesamte Mittel, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren bevorzugten Ausführungsform kann die Wirkung der erfindungsgemäßen Wirkstoffkombination durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls^{®} PGPH)
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Als Perlglanzwachse kommen beispielsweise in Frage: Alkylenglycolester; Fettsäurealkanolamide; Partialglyceride; Ester von mehrwertigen, gegebenenfalls hydroxysubstituierte Carbonsäuren mit Fett-alkoholen mit 6 bis 22 Kohlenstoffatomen; Fettstoffe, wie beispielsweise Fettalkohole, Fettketone, Fettaldehyde, Fettether und Fettcarbonate, die in Summe mindestens 24 Kohlenstoffatome aufweisen; Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimer-diol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

Es ist bevorzugt, dass die erfindungsgemäßen Mittel als Ölkörper - bezogen auf ihr Gewicht - 0,01 bis 10 Gew.-% eines nichtflüchtigen, unlöslichen Silikons enthalten. Insbesondere bevorzugt sind Dimethicone mit einer Viskosität im Bereich von 2.000 bis 300.000 cSt.

In einer weiteren bevorzugten Ausführungsform der Erfindung wird die Wirkstoffkombination als Haarspülung oder Haarkur konfektioniert.

Diese enthalten neben der Basis Wasser quartäre Ammonioumverbindungen (wie sie in den vorangegangenen Abschnitten bereits beschrieben worden sind), niedere Alkohole, Proteinhydrolysate, Lösungsvermittler, kationische und/oder nichtionische Polymere, Ölkörper, organische Säuren, Mittel zur Einstellung der Viskosität und Parfümöle.

In einer weiteren bevorzugten Ausführungsform der Erfindung wird die Wirkstoffkombination als Haarfestiger konfektioniert.

Diese enthalten neben Wasser niedere Alkohole, Tenside, bevorzugt nichtionische Tenside (wie sie in den vorangegangenen Abschnitten bereits beschrieben worden sind), Stabilisatoren, kationische, nichtionische und/oder anionische Polymere, Mittel zur Einstellung des pH-Wertes sowie, falls als Schaumfestiger formuliert, Treibmittel.

Bei den anionischen Polymeren, welche die Wirkung der erfindungsgemäßen Wirkstoffkombination unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze^{®} QM im Handel erhältlich.

Weiterhin können als Polymere zur Steigerung der Wirkung der erfindungsgemäßen Wirkstoffkombination amphotere Polymere als Bestandteil eingesetzt werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (VI),

   R²²-CH=CR²³-CO-Z-(CₙH₂ₙ)-N⁽⁺⁾R²⁴R²⁵R²⁶ A⁽⁻⁾ (IV)

   in der R²² und R²³ unabhängig voneinander stehen für Wasserstoff oder eine Methylgruppe und R²⁴, R²⁵ und R²⁶ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (VII),

   R²⁷-CH=CR²⁸-COOH (VII)

   in denen R²⁷ und R²⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R²⁴, R²⁵ und R²⁶ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

In einer weiteren bevorzugten Ausführungsform der Erfindung wird die Wirkstoffkombination als Hautcreme konfektioniert.

Weiterhin kann in einer bevorzugten Ausführungsform der Erfindung die Wirkung des erfindungsgemäßen Mittels durch UV-Filter (I) gesteigert werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315 nm)- oder im UVC(< 280 nm)-Bereich liegt. UV- Filter mit einem Absorptionsmaximum im UVB-Bereich, insbesondere im Bereich von etwa 280 bis etwa 300 nm, sind besonders bevorzugt.
Die erfindungsgemäß verwendeten UV-Filter können beispielsweise ausgewählt werden aus substituierten Benzophenonen, p- Aminobenzoesäureestern, Diphenylacrylsäureestern, Zimtsäureestern, Salicylsäureestern, Benzimidazolen und o-Aminobenzoesäureestern.
Beispiele für erfindungsgemäß verwendbar UV-Filter sind 4- Aminobenzoesäure, N,N,N-Trimethyl-4-(2-oxoborn-3-ylidenmethyl)anilin- methylsulfat, 3,3,5-Trimethyl-cyclohexylsalicylat (Homosalate), 2- Hydroxy-4-methoxy-benzophenon (Benzophenone-3; Uvinul®M 40, Uvasorb®MET, Neo Heliopan®BB, Eusolex®4360), 2-Phenylbenzimidazol-5-sulfonsäure und deren Kalium-, Natrium- und Triethanolaminsalze (Phenylbenzimidazole sulfonic acid; Parsol®HS; Neo Heliopan®Hydro), 3,3'-(1,4- Phenylendimethylen)-bis(7,7-dimethyl-2-oxo-bicyclo-[2.2.1]hept-1-yl- methan-sulfonsäure) und deren Salze, 1-(4-tert.-Butylphenyl)-3-(4- methoxyphenyl)-propan-1,3-dion (Butyl methoxydibenzoylmethane; Parsol®1789, Eusolex®9020), α-(2-Oxoborn-3-yliden)-toluol-4- sulfonsäure und deren Salze, ethoxylierte 4-Aminobenzoesäure-ethylester (PEG-25 PABA; Uvinul®P 25), 4-Dimethylaminobenzoesäure-2-ethylhexylester (Octyl Dimethyl PABA; Uvasorb®DMO, Escalol®507, Eusolex®6007), Salicylsäure-2-ethylhexylester (Octyl Salicylat; Escalol®587, Neo Heliopan®OS, Uvinul®O18), 4-Methoxyzimtsäure-isopentylester (Isoamyl p- Methoxycinnamate; Neo Heliopan®E 1000), 4-Methoxyzimtsäure-2-ethylhexyl- ester (Octyl Methoxycinnamate; Parsol®MCX, Escalol®557, Neo Heliopan®AV), 2-Hydroxy-4- methoxybenzophenon-5-sulfonsäure und deren Natriumsalz (Benzophenone-4; Uvinul®MS 40; Uvasorb®S 5), 3-(4'-Methylbenzyliden)-D,L- Campher (4-Methylbenzylidene camphor; Parsol®5000, Eusolex®6300), 3- Benzyliden-campher (3-Benzylidene camphor), 4-Isopropylbenzylsalicylat, 2, 4,6-Trianilino-(p-carbo-2'-ethylhexyl-1'-oxi)-1,3,5-triazin, 3-Imidazol-4- yl-acrylsäure und deren Ethylester, Polymere des N-{(2 und 4)-[2-oxoborn- 3- ylidenmethyl]benzyl}-acrylamids, 2,4-Dihydroxybenzophenon (Benzophenone-1; Uvasorb®20 H, Uvinul®400), 1,1'- Diphenylacrylonitrilsäure-2-ethylhexyl-ester (Octocrylene; Eusolex®OCR, Neo Heliopan®Type 303, Uvinul®N 539 SG), o-Aminobenzoesäure-menthylester (Menthyl Anthranilate; Neo Heliopan®MA), 2,2',4,4'- Tetrahydroxybenzophenon (Benzophenone-2; Uvinul®D-50), 2,2'-Dihydroxy-4, 4'-dimethoxybenzophenon (Benzophenone-6), 2,2'-Dihydroxy-4,4'-dimethoxybenzophenon-5-natriumsulfonat und 2-Cyano-3,3-diphenylacrylsäure- 2'-ethylhexylester. Bevorzugt sind 4-Aminobenzoesäure, N,N,N-Trimethyl-4- (2-oxoborn-3-ylidenmethyl)anilin-methylsulfat, 3,3,5- Trimethyl- cyclohexylsalicylat, 2-Hydroxy-4-methoxy-benzophenon, 2- Phenylbenzimidazol-5-sulfonsäure und deren Kalium-, Natrium- und Triethanolaminsalze, 3,3'-(1,4- Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α- (2- Oxoborn-3-yliden)-toluol-4-sulfonsäure und deren Salze, ethoxylierte 4- Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2- Hydroxy-4-methoxybenzophenon-5- sulfonsäure und deren Natriumsalz, 3-(4'-Methylbenzyliden)-D,L-Campher, 3- Benzyliden-campher, 4-lsopropylbenzylsalicylat, 2,4,6-Trianilino-(p-carbo- 2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}- acrylamid. Erfindungsgemäß ganz besonders bevorzugt sind 2-Hydroxy-4- methoxy-benzophenon, 2- Phenylbenzimidazol-5-sulfonsäure und deren Kalium- , Natrium- und Triethanolaminsalze, 1-(4-tert.-Butylphenyl)-3-(4- methoxyphenyl)-propan-1,3-dion, 4-Methoxyzimtsäure-2- ethylhexyl-ester und 3-(4'-Methylbenzyliden)-D,L-Campher.
Bevorzugt sind solche UV-Filter, deren molarer Extinktionskoeffizient am Absorptionsmaximum oberhalb von 15 000, insbesondere oberhalb von 20000, liegt. Weiterhin wurde gefunden, daß bei strukturell ähnlichen UV- Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20°C zu nicht mehr als 1 Gew.-%, insbesondere zu nicht mehr als 0,1 Gew.- %, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.
Gemäß einer weiteren Ausführungsform der Erfindung sind solche UV- Filter bevorzugt, die eine kationische Gruppe, insbesondere eine quartäre Ammoniumgruppe, aufweisen. Diese UV-Filter weisen die allgemeine Struktur U-Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV- Filters durch eine kationische Gruppe Q, insbesondere mit einer quartären Aminofunktion, ersetzt wird.
Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N- Dimethylaminobenzoesäureamid ableiten, sind erfindungsgemäß bevorzugt.
Die Strukturteile U können prinzipiell so gewählt werden, daß das Absorptionsmaximum der UV-Filter sowohl im UVA(315-400 nm)-, als auch im UVB(280-315 nm)- oder im UVC(< 280 nm)-Bereich liegen kann. UV-Filter mit einem Absorptionsmaximum im UVB-Bereich, insbesondere im Bereich von etwa 280 bis etwa 300 nm, sind besonders bevorzugt.
Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.
Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.
Vorteilhafterweise hat die Gruppe Q die allgemeine Struktur - (CH₂)ₓN⁺R¹R²R³ X⁻, in der x steht für eine ganze Zahl von 1 bis 4, R¹ und R² unabhängig voneinander stehen für C₁₋₄- Alkylgruppen, R³ steht für eine C ₁₋₂₂-Alkylgruppe oder eine Benzylgruppe und X⁻ für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen. Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat. Zwei bevorzugte UV-Filter mit kationischen Gruppen sind die als Handelsprodukte erhältlichen Verbindungen Zimtsäureamidopropyl- trimethylammoniumchlorid (Incroquat®UV- 283) und Dodecyl- dimethylaminobenzamidopropyldimethylammoniumtosylat (Escalol® HP 610).
Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV- Filters mit mindestens einem UV-Filter mit einer kationischen Gruppe bevorzugt.
Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt.

Schließlich läßt sich die Wirkung des erfindungsgemäßen Mittels auch durch den kombinierten Einsatz mit Pflanzenextrakten (L) steigern.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e. V. (IKW), Frankfurt, beginnenden Tabelle aufgeführt sind.
Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.
Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.
Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1 : 10 bis 10 : 1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch.
Weiterhin kann es bevorzugt sein, in den erfindungsgemäßen Mitteln Mischungen aus mehreren, insbesondere aus zwei, verschiedenen Pflanzenextrakten einzusetzen. Zusätzlich kann es sich als vorteilhaft erweisen, wenn neben dem erfindungsgemäßen Wirkstoffkomplex (A) Penetrationshilfsstoffe und/oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2- Propandiol, 1,2-Pentandiol, 1,2-Hexandiol, 1,2-Dodecandiol, 1,3- Propandiol, 1,6-Hexandiol, 1,5-Pentandiol, 1,4-Butandiol.

Neben dem erfindungsgemäß zwingend erforderlichen Komponenten und den weiteren, oben genannten bevorzugten Komponenten können die erfindungsgemäßen Mittel prinzipiell alle weiteren, dem Fachmann für solche kosmetischen Mittel bekannten Komponenten enthalten.
Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n- decylether, Di-n-nonylether, Di-n-undecylether und Di-n- dodecylether, n- Hexyl-n-octylether, n-Octyl-n-decylether, n-Decyl-n-undecylether, n- Undecyl-n-dodecylether und n-Hexyl-n-Undecylether sowie Di-tert- butylether, Di-isopentylether, Di-3-ethyldecylether, tert.-Butyl-n-octylether, iso-Pentyl-n-octylether und 2-Methyl-pentyl-n-octylether,
- Fettalkohole, insbesondere lineare und/oder gesättigte Fettalkohole mit 8 bis 30 C-Atomen,
- Monoester von C8 bis C30-Fettsäuren mit Alkoholen mit 6 bis 24 C-Atomen,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie
- Phospholipide, beispielsweise Sojalecithin, Ei- Lecithin und Kephaline,
- quaternierte Amine wie Methyl-1-alkylamidoethyl-2- alkylimidazoliniummethosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β- Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α- Mercaptoethansulfonsäure,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die oben genannte Monographie von K. H. Schrader verwiesen.

### Beispiele:

### 1) Haarpflegeshampoo

| | Gew.-% |
|---|---|
| Texapon^{®1} NSO | 40,0 |
| Dehyton^{®2} G | 6,0 |
| Polymer JR^{®3} 400 | 0,4 |
| Cetiol^{®4} HE | 0,8 |
| Lamesoft^{®5} PO 65 | 4,0 |
| Gluadin^{®6} WQT | 2,5 |
| Gluadin^{®7} W 20 | 0,5 |
| Panthenol (50%) | 0,3 |
| Citronensäure | 2,5 |
| Pantolacton | 1,0 |
| Natriumbenzoat | 0,5 |
| Parfüm | 0,4 |
| NaCl | 0,5 |
| Polymer SL | 0,2 |
| Wasser | ad 100 |

### 2) Haarspülung

| | Gew.-% |
|---|---|
| Eumulgin^{®8} B2 | 0,3 |
| Cetyl/Stearylalkohol | 3,3 |
| Isopropylmyristat | 0,5 |
| Dehyquart^{®9} A-CA | 2,0 |
| Salcare^{®10} SC 96 | 1,0 |
| Citronensäure | 1,1 |
| Gluadin^{®11} WQ | 2,0 |
| Pantolacton | 1,0 |
| Phenonip^{®12} | 0,8 |
| Polymer SL | 0,1 |
| Wasser | ad 100 |

### 3) Haarkur

| | Gew.-% |
|---|---|
| Sepigel^{®14} 305 | 4,0 |
| Synthalen^{®15} K | 0,25 |
| Dow Corning^{®16} 1403 Fluid | 1,0 |
| Natronlauge (50% Standard) | 0,15 |
| Gluadin^{®17} WLM | 0,5 |
| Luviskol^{®18} K30 | 0,1 |
| Polymer^{®3} JR 400 | 0,3 |
| Parfüm | 0,2 |
| Dehyquart^{®19} F 75 | 0,2 |
| Gluadin^{®11} WQ | 0,25 |
| Gafquat^{®20} 755 N | 1,5 |
| Ethanol 96% | 10,0 |
| Polymer SL | 0,1 |
| Wasser | ad 100 |

### Handelsprodukte:

| | |
|---|---|
| 1 | Natriumlaurylethersulfat; AS: 26,5 - 27%, Cognis |
| 2 | Kokosfettsäureamidoethyl-N-2-hydroxyethylglycin; INCI: Disodium Cocoamphodiacetate; WAS 29 - 31 %; Cognis |
| 3 | Hydroxyethylcellulose, quaternisiert; INCI: Polyquaternium-10; Amerchol |
| 4 | Kokosmonoglycerid, ethoxyliert (7 EO); PEG-7 Glyceryl Cocoate; Cognis |
| 5 | Alkylpolyglucosid, Ölsäuremonoglycerid-Gemisch; INCI: Coco-Glucoside, Glyceryl Oleate; FK: 65 - 70%; Cognis |
| 6 | INCI: Hydroxypropyltrimonium hydrolyzed wheat protein; FK: 26 - 30%; Cognis |
| 7 | Weizenproteinhydrolysat; INCI: Hydrolyzed wheat protein; FK: 20%; Cognis |
| 8 | C₁₆-C₁₈-Fettalkohole, ethoxyliert (20EO); INCI: Ceteareth-20; Cognis |
| 9 | Trimethylhexadecylammoniumchlorid; INCI:Cetrimonium Chloride; 24-26%; Cognis |
| 10 | INCI. Polyquaternium-37; AS: 50%; Ciba |
| 11 | INCI: Laurdimonium Hydroxypropyl Hydrolyzed Wheat Protein; FK: 31 - 35%; Cognis |
| 12 | INCI: Phenoxyethanol, Methylparaben, Ethylparaben, Propylparaben, Butylparaben; AS: 27 - 28,3%; Clariant |

| | |
|---|---|
| 14 | INCI: Polyacrylamide, C13-14 Isoparaffin, Laureth-7; AS: 45 - 49%; Seppic |
| 15 | Polyacrylsäure; INCI: Carbomer; 3V Sigma |
| 16 | INCI: Dimethicone, Dimethiconol; Dow Corning |
| 17 | Weizenproteinhydrolysat; INCI: Hydrolyzed Wheat Protein; FK. 21 - 24%; Cognis |
| 18 | Polyvinylpyrrolidon; INCI: PVP; FK: 95 - 100%; BASF |
| 19 | INCI: Distearoylethyl Hydroxyethylmonium Methosulfate; 65 - 72%; Cognis |
| 20 | Vinylpyrrolidon Dimethylaminoethylmethacrylat Copolymer, Diethylsulfat quaternisiert; INCI: Polyquaternium-11; FK: 19 - 21%; ISP |

## Patentansprüche

1. Reinigungs- und -konditioniermittel für Haut und Haare, enthaltend - bezogen auf sein Gewicht -
a) 0,001 bis 10 Gew.-% mindestens eines quaternisierten Hydroxyethylcellulosepolymers, das mit Trimethylommoniumgruppen Kationisch substituiert und mit Dimethyldodecylammoniumgruppen hydrophob modifiziert wurde, sowie
b) mindestens ein Proteinhydrolysat.

2. Reinigungs- und -konditioniermittel für Haut und Haare nach Anspruch 1, **dadurch gekennzeichnet, dass** das Polymer a) einen kationischen Substitutionsgrad von 0,15 bis 0,25 aufweist.

3. Reinigungs- und -konditioniermittel für Haut und Haare nach einem der Ansprüche 1 oder 2, **dadurch gekennzeichnet, dass** das Polymer a) einen hydrophoben Substitutionsgrad (HS) von HS ≤ 0,01 aufweist.

4. Reinigungs- und -konditioniermittel für Haut und Haare nach einem der Ansprüche 1 bis 3, **dadurch gekennzeichnet, dass** das Polymer a) in einer dreiprozentigen, wässrigen Lösung bei einer Temperatur von 20 °C und einer Scherrate von 0,1/s eine Viskosität von 500 bis 900 Pas aufweist (gemessen mit dem schubspannungsgesteuerten Rotationsviskosimeter AR-1000N der Firma Thermal Analysis, Platte-Kegel Messsystem mit 4 cm Durchmesser und einem Winkel von 1°, Spaltweite: 32).

5. Reinigungs- und Konditioniermittel für Haut und Haare nach einem der Ansprüche 1 bis 4, **dadurch gekennzeichnet, dass** das Polymer a) in einer dreiprozentigen, wässrigen Lösung bei einer Temperatur von 20 °C eine Fließgrenze von 100-200 Pa aufweist (gemessen im Oszillationsversuch mit einer konstanten Frequenz von 1 Hz und ansteigender Amplitude mit dem schubspannungsgesteuerten Rotationsviskosimeter AR-1000N der Firma Thermal Analysis, Platte-Kegel Messsystem mit 4 cm Durchmesser und einem Winkel von 1°, Spaltweite: 32).

6. Reinigungsmittel für Haut und Haare nach einem der Ansprüche 1 bis 5, **dadurch gekennzeichnet, dass** es - bezogen auf sein Gewicht- 0,1 bis 50 Gew.-% eines Tensidsystems, ausgewählt aus anionischen, amphoteren, zwitterionischen und nichtionischen Tensiden sowie aus Gemischen dieser Tensidklassen enthält.

7. Reinigungsmittel für Haut und Haare nach Anspruch 6, **dadurch gekennzeichnet, dass** das Tensidsystem Tenside aus den Gruppen der Alkylethersulfate, Aminoxide, Alkylpolyglycoside, Betaine, Alkyletherphosphate, Acyltaurine, Sulfobernsteinsäureester, Alkylethercarboxylate und/oder der Sorbitanester enthält.

8. Reinigungsmittel für Haut und Haare nach Anspruch 7, **dadurch gekennzeichnet, dass** das Tensidsystem, Alkylethersulfate, Aminoxide, Alkylpolyglucoside und/oder Betaine enthält.

9. Konditioniermittel für Haut und Haare nach einem der Ansprüche 1 bis 5, **dadurch gekennzeichnet, dass** es im Falle des Konditioniermittels - bezogen auf sein Gewicht - 0,1 bis 30 Gew.-% mindestens eines kationischen Tensids enthält.

10. Reinigungs- und -konditioniermittel für Haut und Haare nach einem der Ansprüche 1 bis 9, **dadurch gekennzeichnet, dass** es die Vitamine, Vitaminvorstufen oder Vitaminderivate der Gruppen A, B, C und/oder E und/oder Lysin, Isoleucin, Tyrosin, Cystein, Cystin, Alanin, Serin, Tryptophan, Glutaminsäure, Threonin, Valin, Glycin, Prolin, Arginin und/oder Taurin und/oder Citronensäure, Bernsteinsäure, Salicylsäure, Milchsäure und/oder 4-Hydroxybenzoesäure und/oder kationische Polymere enthält, die ausgewählt sind aus Polyquaternium-37 und/oder Polymeren auf der Basis von Cellulose oder Hydroxypropyl Guar.

11. Reinigungs- und -konditioniermittel für Haut und Haare nach einem der Ansprüche 1 bis 10, **dadurch gekennzeichnet, dass** es - bezogen auf sein Gewicht - 0,01 bis 10 Gew.-% eines nichtflüchtigen, unlöslichen Silikons enthält.

12. Reinigungs- und -konditioniermittel für Haut und Haare nach Anspruch 11, **dadurch gekennzeichnet, dass** es Dimethicon mit einer Viskosität im Bereich von 2.000 bis 300.000 cSt enthält.

## Claims

1. A cleaning and conditioning agent for skin and hair, containing, relative to its weight,
a) 0.001 to 10 wt.% of at least one quaternised hydroxyethylcellulose polymer which has been cationically substituted with trimethylammonium groups and hydrophobically modified with dimethyldodecylammonium groups, and
b) at least one protein hydrolysate.

2. A cleaning and conditioning agent for skin and hair according to claim 1, **characterised in that** polymer a) has a cationic degree of substitution of 0.15 to 0.25.

3. A cleaning and conditioning agent for skin and hair according to either one of claims 1 or 2, **characterised in that** polymer a) has a hydrophobic degree of substitution (HS) of HS ≤ 0.01.

4. A cleaning and conditioning agent for skin and hair according to any one of claims 1 to 3, **characterised in that**, in a three percent aqueous solution at a temperature of 20°C and a shear rate of 0.1/s, polymer a) has a viscosity of 500 to 900 Pa·s (measured with the AR-1000N shear stress-controlled rotational viscometer from Thermal Analysis, plate-and-cone measurement system with 4 cm diameter and an angle of 1°, gap size: 32).

5. A cleaning and conditioning agent for skin and hair according to any one of claims 1 to 4, **characterised in that**, in a three percent aqueous solution at a temperature of 20°C, polymer a) has a yield point of 100-200 Pa (measured by oscillation testing at a constant frequency of 1 Hz and an increasing amplitude with the AR-1000N shear stress-controlled rotational viscometer from Thermal Analysis, plate-and-cone measurement system with 4 cm diameter and an angle of 1°, gap size: 32).

6. A cleaning and conditioning agent for skin and hair according to any one of claims 1 to 5, **characterised in that** the cleaning agent, relative to its weight, contains 0.1 to 50 wt.% of a surfactant system selected from anionic, amphoteric, zwitterionic and nonionic surfactants and from mixtures of these classes of surfactants.

7. A cleaning and conditioning agent for skin and hair according to claim 6, **characterised in that** the surfactant system contains surfactants from the groups of alkyl ether sulfates, amine oxides, alkyl polyglycosides, betaines, alkyl ether phosphates, acyl taurines, sulfosuccinic acid esters, alkyl ether carboxylates and/or sorbitan esters.

8. A cleaning and conditioning agent for skin and hair according to claim 7, **characterised in that** the surfactant system contains alkyl ether sulfates, amine oxides, alkyl polyglucosides and/or betaines.

9. A cleaning and conditioning agent for skin and hair according to any one of claims 1 to 5, **characterised in that** the conditioner contains, relative to its weight, 0.1 to 30 wt.% of at least one cationic surfactant.

10. A cleaning and conditioning agent for skin and hair according to any one of claims 1 to 9, **characterised in that** it furthermore contains vitamins, vitamin precursors or vitamin derivatives of groups A, B, C and/or E and/or lysine, isoleucine, tyrosine, cysteine, cystine, alanine, serine, tryptophan, glutamic acid, threonine, valine, glycine, proline, arginine and/or taurine and/or citric acid, succinic acid, salicylic acid, lactic acid and/or 4-hydroxybenzoic acid and/or cationic polymers which are selected from Polyquaterium 37 and/or polymers based on cellulose or hydroxypropyl guar.

11. A cleaning and conditioning agent for skin and hair according to any one of claims 1 to 10, **characterised in that** it contains, relative to its weight, 0.01 to 10 wt.% of a non-volatile, insoluble silicone.

12. A cleaning and conditioning agent for skin and hair according to claim 11, **characterised in that** it contains dimethicone with a viscosity in the range from 2,000 to 300,000 cSt.

## Revendications

1. Agent de nettoyage et de revitalisation pour la peau et les cheveux contenant - rapporté à son poids -
a) de 0,001 à 10 % en poids d'au moins un polymère quaternisé d'hydroxyéthylcellulose, qui a été soumis à une substitution cationique avec des groupes de triméthylammonium et à une modification hydrophobe avec des groupes de diméthyldodécylammonium, ainsi que
b) au moins un hydrolysat protéique.

2. Agent de nettoyage et de revitalisation pour la peau et les cheveux selon la revendication 1, **caractérisé en ce que** le polymère a) présente un degré de substitution cationique de 0,15 à 0,25.

3. Agent de nettoyage et de revitalisation pour la peau et les cheveux selon l'une quelconque des revendications 1 ou 2, **caractérisé en ce que** le polymère a) présente un degré de substitution hydrophobe HS ≤ 0,01.

4. Agent de nettoyage et de revitalisation pour la peau et les cheveux selon l'une quelconque des revendications 1 à 3, **caractérisé en ce que** le polymère a) présente une viscosité de 500 à 900 Pas dans une solution aqueuse à 3 % à une température de 20 °C et à un taux de cisaillement de 0,1/s (la viscosité étant mesurée avec le viscosimètre rotatif réglé en ce qui concerne la contrainte de cisaillement AR-1000N de la firme Thermal Analysis, système de mesure du type cône/plan possédant un diamètre de 4 cm et un angle de 1°, largeur de fente : 32).

5. Agent de nettoyage et de revitalisation pour la peau et les cheveux selon l'une quelconque des revendications 1 à 4, **caractérisé en ce que** le polymère a) présente une limite d'élasticité de 10D à 200 Pa dans une solution aqueuse à 3 % à une température de 20 °C (la limite d'élasticité étant mesurée dans un essai d'oscillation avec une fréquence constante de 1 Hz et une amplitude croissante avec le viscosimètre rotatif réglé en ce qui concerne la contrainte de cisaillement AR-1000N de la firme Thermal Analysis, système de mesure du type cône/plan possédant un diamètre de 4 cm et un angle de 1°, largeur de fente : 32).

6. Agent de nettoyage et de revitalisation pour la peau et les cheveux selon l'une quelconque des revendications 1 à 5, **caractérisé en ce qu'**il contient - rapporté à son poids - de 0,1 à 50 % en poids d'un système d'agents tensioactifs choisis parmi des agents tensioactifs anioniques, amphotères, zwitterioniques et non ioniques, et des mélanges de ces classes d'agents tensioactifs.

7. Agent de nettoyage et de revitalisation pour la peau et les cheveux selon la revendication 6, **caractérisé en ce que** le système d'agents tensioactifs contient des agents tensioactifs choisis parmi les groupes des alkyléthersulfates, des aminoxydes, des alkylpolyglycosides, des bétaïnes, des alkylétherphosphates, des acyltaurines, des esters de l'acide sulfosuccinique, des alkyléthercarboxylates et/ou des esters de sorbitanne.

8. Agent de nettoyage et de revitalisation pour la peau et les cheveux selon la revendication 7, **caractérisé en ce que** le système d'agents tensioactifs contient des alkyléthersulfates, des aminoxydes, des alkylpolyglucosides et/ou des bétaïnes.

9. Agent de nettoyage et de revitalisation pour la peau et les cheveux selon l'une quelconque des revendications 1 à 5, **caractérisé en ce qu'**il contient, dans le cas de l'agent de revitalisation - rapporté à son poids - de 0,1 à 30 % en poids d'au moins un agent tensioactif cationique.

10. Agent de nettoyage et de revitalisation pour la peau et les cheveux selon l'une quelconque des revendications 1 à 9, **caractérisé en ce qu'**il contient les vitamines, les précurseurs de vitamines ou les dérivés de vitamines des groupes A, B, C et/ou E et/ou de la lysine, de l'isoleucine, de la tyrosine, de la cystéine, de la cystine, de l'alanine, de la sérine, du tryptophane, de l'acide glutamique, de la thréonine, de la valine, de la glycine, de la proline, de l'arginine et/ou de la taurine et/ou de l'acide citrique, de l'acide succinique, de l'acide salicylique, de l'acide lactique et/ou de l'acide 4-hydroxybenzoique et/ou des polymères cationiques qui sont choisis parmi le Polyquaternium-37 et/ou des polymères à base de cellulose ou d'hydroxypropyl-guar.

11. Agent de nettoyage et de revitalisation pour la peau et les cheveux selon l'une quelconque des revendications 1 à 10, **caractérisé en ce qu'**il contient - rapporté à son poids - de 0,01 à 10 % en poids d'une silicone insoluble non volatile.

12. Agent de nettoyage et de revitalisation pour la peau et les cheveux selon la revendication 11, **caractérisé en ce qu'**il contient du Dimethicone possédant une viscosité dans la plage de 2000 à 300.000 cSt.
